# EUROPÄISCHE PATENTSCHRIFT

(11) **EP 4 337 116 B1**
(45) Veröffentlichungstag und Bekanntmachung des Hinweises auf die Patenterteilung: **23.10.2024**
(21) Anmeldenummer: 22728475.9
(22) Anmeldetag: 10.05.2022
(51) Int. Cl.: A61B 17/34

(54) **KANÜLENANORDNUNG**
CANNULA ASSEMBLY
ENSEMBLE CANULE

(30) Priorität: 11.05.2021 DE 102021204780
(43) Veröffentlichungstag der Anmeldung: 20.03.2024
(73) Patentinhaber: B. Braun Melsungen AG, 34212 Melsungen (DE)
(72) Erfinder: GROSSER, Lars, 34212 Melsungen (DE); WEISS, André, 34302 Guxhagen (DE); RIEMANN, Thomas, 37247 Großalmerode (DE)
(74) Vertreter: Patentanwälte Ruff, Wilhelm, Beier, Dauster & Partner mbB
(86) Internationale Anmeldenummer: PCT/EP2022/062538
(87) Internationale Veröffentlichungsnummer: WO 2022/238356

(56) Entgegenhaltungen:
- WO-A1-2020/231455
- DE-A1- 2 109 608
- DE-A1- 3 221 244
- US-A1- 2009 259 126
- US-A1- 2015 297 213

## Beschreibung

Die Erfindung betrifft eine Kanülenanordnung, aufweisend ein längserstrecktes Kanülenrohr mit einer proximalen Eintrittsöffnung, einer distalen Austrittsöffnung, einem zwischen der Eintrittsöffnung und der Austrittsöffnung durchgängig längserstreckten Lumen und mit einer die Austrittsöffnung berandenden angeschärften Kanülenspitze, wobei das Kanülenrohr eingerichtet ist zur Aufnahme und Führung eines biegeflexiblen medizinischen Funktionsstrangs, der in das Lumen einführbar und entlang des Lumens beweglich ist. Kanülenanordnungen sind in der medizinischen Praxis allgemein bekannt und werden unter anderem zur Punktion von menschlichem oder tierischem Körpergewebe sowie zum anschließenden Einbringen und/oder Entnehmen von Flüssigkeiten verwendet.

Zu diesem Zweck weisen übliche Kanülenanordnungen ein längserstrecktes Kanülenrohr mit einer proximalen Eintrittsöffnung und einer distalen Austrittsöffnung auf. Zwischen der Ein- und der Austrittsöffnung ist ein durchgängig längserstrecktes Lumen vorgesehen. Die Austrittsöffnung wird von einer angeschärften Kanülenspitze berandet, die oftmals auch als Anschliff oder Kanülenauge bezeichnet wird. Die angeschärfte Kanülenspitze erleichtert das Punktieren des Körpergewebes.

Überdies sind unterschiedliche medizinische Anwendungsfälle bekannt, in welchen das Kanülenrohr als Mandrin für einen in den Körper einzuführenden medizinischen Funktionsstrang dient. Der medizinische Funktionsstrang kann beispielsweise eine längserstreckte Sonde, ein Lichtleiter, ein Führungsdraht oder dergleichen sein.

Die US 2015/297213 A1 offenbart eine medizinische Kanülenanordnung zur perkutanen Fixierung des Magens eines Patienten. Die Kanülenanordnung weist eine Nadel mit einer Nadelnabe an ihrem proximalen Ende und ein Stilett mit einer Stilettnabe an ihrem proximalen Ende auf. Laschen in der Nadelnabe blockieren die proximal gerichtete Rückzugsbewegung des Stiletts.

Die US 2009/259126 A1 offenbart eine Kanülenanordnung zur Abgabe von Arzneimitteln in den Halsbereich der Wirbelsäule eines Patienten mit einer Kanüle, einer Nabe am proximalen Ende der Kanüle und einem in die Kanüle verschiebbaren Injektionsschlauch. Eine proximal gerichtete Rückzugsbewegung des Injektionsschlauchs wird durch eine interne Konstruktion der Nabe blockiert.

Die DE 32 21 244 A1 offenbart eine Vorrichtung zum Einführen eines langgestreckten Gegenstandes, insbesondere eines Katheters, einer Sonde oder dergleichen, in eine Körperhöhle, bestehend aus einem gegebenenfalls längsteilbaren Führungsstück mit einem an beiden Enden offenen Durchlaß und einer in den Durchlaß eingebauten Rückzugsperre für den langgestreckten Gegenstand.

DE 21 09 608 A1 offenbart eine Vorrichtung zur Einführung eines Katheters in ein Blutgefäß und insbesondere zum Bewegen eines Katheters in einem Blutgefäß.

Die WO2020231455 A1 offenbart ein Gefäßzugangsgerät mit integrierten Sicherheitsvorrichtungen, die die Migration von Führungsdrahten in den Körper des Patienten verhindern.

Ein wichtiger Anwendungsfall ist die Anlage zentralvenöser Katheter nach der sogenannten Seldinger-Technik. Diese sieht insbesondere vor, dass ein Führungsdraht in die Eintrittsöffnung des Kanülenrohrs eingeführt, in distaler Vorschubrichtung durch das Lumen vorgeschoben und aus der Austrittsöffnung ausgeführt wird.

Aufgabe der Erfindung ist es, eine Kanülenanordnung der eingangs genannten Art bereitzustellen, die eine erhöhte Patientensicherheit bietet.

Diese Aufgabe wird durch eine Kanülenanordnung gemäss Anspruch 1 gelöst.

Weitere Ausführungsformen der Erfindung sind in den abhängigen Ansprüchen definiert.

Die Erfindung geht von der Erkenntnis aus, dass der medizinische Funktionsstrang bei einer proximalen Rückzugbewegung, d.h. bei einem Zurückziehen durch das Kanülenrohr, beschädigt oder gar durchtrennt werden kann. Denn bei dem Zurückziehen kann der Funktionsstrang unter Umständen an der die Austrittsöffnung berandenden angeschärften Kanülenspitze zur Anlage gelangen, wobei die angeschärfte Kanülenspitze als eine Art Schneide auf den Funktionsstrang einwirken kann. Dies kann zu schwerwiegenden Komplikationen führen. Unter Umständen kann eine Notfalloperation zum Entfernen des teilweise abgetrennten Funktionsstrangs aus dem Körper notwendig werden. Um alldem entgegenzuwirken, sieht die erfindungsgemäße Lösung die Sperrvorrichtung vor. Die Sperrvorrichtung bewirkt eine Sperrung der Beweglichkeit des Funktionsstrangs in proximaler Richtung, so dass der Funktionsstrang zwar ohne weiteres in distaler Richtung vorgeschoben, jedoch nicht oder lediglich in begrenztem Umfang in proximaler Richtung zurückgezogen werden kann. Hierdurch wird einer Beschädigung des Funktionsstrangs durch die angeschärfte Kanülenspitze entgegenwirkt und letztlich eine verbesserte Patientensicherheit erreicht. Vorzugsweise wirkt die Sperrvorrichtung mechanisch mit dem Funktionsstrang zusammen, so dass auch von einer mechanischen Sperrvorrichtung gesprochen werden kann. Alternativ oder zusätzlich kann die Sperrvorrichtung magnetisch und/oder elektrisch mit dem Funktionsstrang zusammenwirken. Zum Sperren der Beweglichkeit des Funktionsstrangs weist die Sperrvorrichtung das wenigstens eine dem Lumen zugeordnete Sperrelement auf. Das wenigstens eine Sperrelement ist unter Einwirkung des in das Lumen eingeführten Funktionsstrangs und in Abhängigkeit der jeweiligen Bewegungsrichtung zwischen der Freigabe- und der Sperrstellung selbsttätig überführbar. Das Überführen zwischen der Freigabe- und der Sperrstellung kann eine elastische Deformation, insbesondere Biegedeformation, und/oder eine, insbesondere translatorische und/oder rotatorische, Starrkörperbewegung des wenigstens einen Sperrelements umfassen. In der Sperrstellung wirkt das wenigstens eine Sperrelement auf den Funktionsstrang, so dass eine proximale Verlagerung oder jedenfalls eine weitergehende proximale Verlagerung des Funktionsstrangs durch das Lumen unterbunden ist. Vorzugsweise wirkt das wenigstens eine Sperrelement in der Sperrstellung kraft- und/oder formschlüssig auf eine Außenfläche des Funktionsstrangs ein. Selbsttätig meint, dass das Überführen bzw. der Wechsel zwischen der Freigabe- und der Sperrstellung je nach Bewegungsrichtung des Funktionsstrangs gleichsam automatisch und insbesondere ohne gesonderten Eingriff von außen erfolgt. Vorzugsweise ist das wenigstens eine Sperrelement in der Freigabestellung relativ zu dem Lumen radial nach außen verlagert. Weiter vorzugsweise ist das wenigstens eine Sperrelement in der Sperrstellung relativ zu dem Lumen radial nach innen verlagert. In einem Anwendungszustand derKanülenanordnung, in welchem der medizinische Funktionsstrang in das Lumen eingeführt ist, wirkt das wenigstens eine Sperrelement vorzugsweise mechanisch mit der Außenfläche des Funktionsstrangs zusammen. Vorzugsweise wirkt das wenigstens eine Sperrelement in der Sperrstellung proximal entlang einer Längsachse des Lumens - und damit entlang einer Längsachse des Funktionsstrangs - kraft- und/oder formschlüssig auf den Funktionsstrang, genauer: dessen Außenfläche und/oder Mantelfläche, ein. In der Freigabestellung ist diese Einwirkung aufgehoben. Der medizinische Funktionsstrang ist nicht Bestandteil der erfindungsgemäßenKanülenanordnung. Ausgestaltungen der erfindungsgemäßen Kanülenanordnung können einen bzw. den medizinischen Funktionsstrang umfassen. Der medizinische Funktionsstrang ist längserstreckt und biegeflexibel und kann insbesondere ein medizinischer Draht, wie beispielsweise ein Führungsdraht zur Katheteranlage, eine medizinische Sonde, ein Lichtleiter, eine Faden- oder Drahtanordnung, die jeweils verzwirnt und/oder verdrillt sein kann, oder dergleichen sein.

In Ausgestaltung der Erfindung ist die Sperrvorrichtung in das Kanülenrohr integriert.

Eine Integration der Sperrvorrichtung in das Kanülenrohr ermöglicht insbesondere einen besonders kompakten Aufbau der Kanülenanordnung. Dabei kann das wenigstens eine Sperrelement insbesondere durch einen Rohrwandabschnitt einer Rohrwandung des Kanülenrohrs gebildet sein. Alternativ kann das wenigstens eine Sperrelement separat von dem Kanülenrohr gefertigt und hiernach in dieses eingesetzt und/oder integriert sein. Eine Integration der Sperrvorrichtung in den Kanülenansatz bietet vergleichsweise mehr Bauraum und eröffnet insoweit weitere konstruktive Freiheitsgrade im Hinblick auf die räumlich-körperliche Gestaltung der Sperrvorrichtung und/oder des wenigstens einen Sperrelements. Der Kanülenansatz kann auch als Kanülenhub bezeichnet werden und ist proximal an dem Kanülenrohr befestigt. Abgesehen von der integrierten Sperrvorrichtung weist der Kanülenansatz vorzugsweise einen üblichen Aufbau auf, so dass insbesondere ein proximal angeordneter Anschlussport zum Anschließen einer fluidführenden Komponente an den Kanülenansatz vorhanden sein kann. Der Anschlussport kann beispielsweise als Luer-Anschluss ausgestaltet sein. Vorzugsweise ist der Kanülenansatz unlösbar mit dem Kanülenrohr zusammengefügt. Bei dieser Ausgestaltung der Erfindung ist der Kanülenansatz Bestandteil der Kanülenanordnung. Das besagte Vorrichtungsgehäuse erlaubt einen besonders flexiblen Einsatz der Sperrvorrichtung. Das Vorrichtungsgehäuse ist proximal an dem Kanülenrohr befestigt und/oder befestigbar. Sofern die Kanülenanordnung einen Kanülenansatz aufweist, ist das Vorrichtungsgehäuse proximal an dem Kanülenansatz befestigt und/oder befestigbar. Die jeweilige Befestigung ist vorzugsweise lösbar gestaltet. Sofern ein Kanülenansatz und/oder ein Vorrichtungsgehäuse vorhanden sind, ist der Funktionsstrang - in einem Anwendungszustand der Kanülenanordnung - durch ein jeweiliges Lumen des Katheteransatzes und/oder des Vorrichtungsgehäuses längserstreckt.

In weiterer Ausgestaltung der Erfindung ist das wenigstens eine Sperrelement eine Sperrklinke, die elastisch federbeweglich und/oder schwenkbeweglich zwischen der Freigabestellung und der Sperrstellung verlagerbar ist und eine in distaler Richtung ausgerichtete Sperrkante aufweist, die bei einer Vorschubbewegung relativ gleitbeweglich mit einer Außenfläche des Funktionsstrangs zusammenwirkt und bei einer Rückzugbewegung reibkraftbedingt gegen die Außenfläche angestellt ist und die Rückzugbewegung kraft- und/oder formschlüssig sperrt. Diese Ausgestaltung der Erfindung ermöglicht einen besonders einfachen Aufbau der Sperrvorrichtung. Die Sperrklinke ist in das Kanülenrohr integriert.

Die Sperrklinke wirkt mittels ihrer Sperrkante mechanisch mit der Außenfläche des Funktionsstrangs zusammen. Die Außenfläche kann auch als Mantelfläche bezeichnet werden. Um eine richtungsabhängige Verlagerung zwischen der Freigabe- und der Sperrstellung zu ermöglichen, ist die Sperrkante in distaler Richtung ausgerichtet. Infolge dieser distalen Ausrichtung bewirkt eine zwischen der Sperrkante und der Außenfläche bewegungsbedingt auftretende Reibkraft entweder eine in Radialrichtung des Lumens nach außen oder nach innen wirkende Kraftkomponente. Die radial nach außen wirkende Kraftkomponente tritt bei der distalen Vorschubbewegung auf. Hierdurch wird die Sperrkante unter Einwirkung der Außenfläche radial nach außen gedrückt. Die radial nach innen wirkende Kraftkomponente tritt bei der proximalen Rückzugbewegung auf. Hierdurch wird die Sperrkante radial nach innen auf den Funktionsstrang, genauer: dessen Außenfläche, gedrückt. Insoweit kann auch von einer Selbstverstärkung und/oder Selbsthemmung gesprochen werden. Durch die radial nach innen wirkende Kraftkomponente kommt zu einem Kraft- und/oder Formschluss zwischen der Sperrkante und der Außenfläche.

In weiterer Ausgestaltung der Erfindung ist die Sperrklinke nach Art einer Biegefeder gestaltet, wobei die Biegefeder einends relativ zu dem Lumen festgelegt ist und andernends die Sperrkante aufweist. Die Biegefeder ist elastisch federbeweglich zwischen der Freigabe- und der Sperrstellung. Hierdurch kann insbesondere auf eine Schwenk- oder ansonsten bewegliche Lagerung der Sperrklinke verzichtet werden. Dies erlaubt eine weitere Vereinfachung der Konstruktion.

In weiterer Ausgestaltung der Erfindung ist die Sperrklinke durch einen zungenförmig freigestellten Rohrwandabschnitt einer Rohrwandung des Kanülenrohres gebildet. Dies erlaubt einen besonders kompakten und einfachen Aufbau der Kanülenanordnung. Zum einen ist bei dieser Ausgestaltung der Erfindung kein gesonderter Bauraum für das wenigstens eine Sperrelement, genauer: die Sperrklinke, erforderlich. Zum anderen sind keine zusätzlichen Bauteile zum Ausbilden der, vorzugsweise mechanischen, Sperrvorrichtung erforderlich. Vielmehr bildet das Kanülenrohr selbst gleichsam die, vorzugsweise mechanische, Sperrvorrichtung. Zu diesem Zweck bildet der zungenförmig freigestellte Rohrwandabschnitt die Sperrklinke aus. Zungenförmig freigestellt meint, dass die Rohrwandung in Radialrichtung mittels eines im weitesten Sinne U-förmigen Einschnitts durchtrennt ist. Der mittels des Einschnitts freigestellte Rohrwandabschnitt weist insoweit im weitesten Sinne eine Zungenform auf. Einends, im Bereich der "Zungenwurzel" ist der Rohrwandabschnitt einstückig zusammenhängend mit der übrigen Rohrwandung. Andernends, im Bereich der "Zungenspitze", weist der Rohrwandabschnitt die Sperrkante auf. Der freigestellte Rohrwandabschnitt ist elastisch federbeweglich zwischen der Freigabe- und der Sperrstellung.

In weiterer Ausgestaltung der Offenbarung ist das wenigstens eine Sperrelement eine um eine Drehachse drehbeweglich gelagerte erste Sperrrolle, deren Außenumfang zum Abwälzen auf dem Funktionsstrang eingerichtet ist, wobei die erste Sperrrolle eine mit der Vorschubbewegung korrespondierende Freilaufdrehrichtung sowie eine entgegengesetzt um die Drehachse gerichtete und mit der Rückzugbewegung korrespondierende Blockierrichtung aufweist. Der Außenumfang der ersten Sperrrolle wirkt radial und/oder tangential mit der Außenfläche des Funktionsstrangs zusammen. Die Drehachse der ersten Sperrrolle ist orthogonal zur Längsachse des Lumens - und damit des Funktionsstrangs - orientiert. Die Ausgestaltung des wenigstens einen Sperrelement als Sperrrolle erlaubt im Vergleich zu einer Ausgestaltung als Sperrklinke insbesondere eine leichtgängigere Vorschubbewegung und eine verminderte Beanspruchung des Funktionsstrangs. Denn der Außenumfang wälzt bei der Vorschubbewegung auf dem Funktionsstrang und/oder dessen Außenfläche ab. Hierbei kommt es lediglich zu einer Rollreibung. Dementgegen tritt bei der Sperrklinke eine Gleitreibung auf. Die auf diese Weise verminderte Reibungseinwirkung erlaubt zum einen die besagte leichtgängige Vorschubbewegung und zum anderen eine verminderte Beanspruchung des Funktionsstrangs. In der Blockierrichtung ist keine Drehbewegung der Sperrrolle möglich. Dementsprechend wirkt der Außenumfang dann unter Sperrung der proximalen Beweglichkeit des Funktionsstrangs kraft- und/oder formschlüssig auf dessen Außenfläche ein.

In weiterer Ausgestaltung der Offenbarung ist die erste Sperrrolle entlang einer ersten Führungsachse an einer ersten Linearführung linearbeweglich geführt verlagerbar, wobei die erste Führungsachse relativ zu einer Längsachse des Lumens geneigt ist, und wobei die erste Sperrrolle unter Einwirkung des Funktionsstrangs entlang der ersten Führungsachse beweglich ist, wodurch der Außenumfang relativ zu dem Funktionsstrang radial nach außen und/oder nach innen verlagerbar ist. Die erste Führungsachse ist relativ zu der Längsachse des Lumens in proximaler Richtung radial nach innen geneigt. Bei einem Zurückziehen des Funktionsstrangs wird die erste Sperrrolle entlang der ersten Führungsachse proximal verlagert, wodurch deren Außenumfang radial nach innen gegen den Funktionsstrang angestellt wird. Dieses Anstellen oder auch Andrücken bewirkt letztlich eine Sperrung der Rückzugbewegung.

In weiterer Ausgestaltung der Offenbarung ist eine zweite Sperrrolle vorhanden und in Radialrichtung des Lumens gegenüber der ersten Sperrrolle angeordnet, wobei die zweite Sperrrolle an einer zweiten Linearführung entlang einer zweiten Führungsachse linearbeweglich geführt verlagerbar ist, wobei die zweite Führungsachse in Bezug auf die Längsachse des Lumens spiegelsymmetrisch zu der ersten Führungsachse orientiert ist. Diese Ausgestaltung der Offenbarung erlaubt eine besonders zuverlässige Sperrung der Rückzugsbewegung. Denn der Funktionsstrang wird bei einem Zurückziehen gleichsam zwischen der ersten Sperrrolle und der zweiten Sperrrolle radial eingeklemmt. Die erste Sperrrolle und die zweite Sperrrolle bilden vorzugsweise ein Sperrrollenpaar. Bei einer weiteren Ausgestaltung sind mehr als zwei Sperrrollen vorhanden und vorzugsweise unter Ausbildung eines Sperrrollenpakets, bevorzugt sternförmig in Bezug auf den Funktionsstrang, angeordnet.

In weiterer Ausgestaltung der Offenbarung ist wenigstens die erste Sperrrolle dreh- und/oder linearbeweglich in dem Vorrichtungsgehäuse aufgenommen. Grundsätzlich denkbar ist zudem eine Integration der ersten Sperrrolle und/oder der zweiten Sperrrolle in das Kanülenrohr oder einen etwaigen Kanülenansatz. Allerdings bietet die Aufnahme in dem Vorrichtungsgehäuse konstruktive Vorteile, da vergleichsweise mehr Bauraum vorgesehen werden kann und übliche Dimensionen des Kanülenrohrs und/oder des Kanülenansatzes beibehalten werden können.

In weiterer Ausgestaltung der Erfindung umfasst die Kanülenanordnung den medizinischen Funktionsstrang. Vorzugsweise ist der medizinische Funktionsstrang ein medizinischer Draht, bevorzugt ein Seldinger-Draht.

Weitere Vorteile und Merkmale der Erfindung ergeben sich aus den Ansprüchen und der nachfolgenden Beschreibung bevorzugter Ausführungsbeispiele der Erfindung, die anhand der Zeichnungen dargestellt sind.
- Fig. 1: zeigt in teilweise abgeschnittener, schematischer Perspektivdarstellung eine aus dem Stand der Technik bekannte Kanülenanordnung im Bereich einer angeschärften Kanülenspitze mitsamt eines medizinischen Funktionsstrangs in Form eines Führungsdrahts,
- Fig. 2: in perspektivischer sowie teilweise aufgeschnittener Darstellung eine Ausführungsform einer nicht beanspruchten Kanülenanordnung mit einem Kanülenrohr, einem Kanülenansatz und einer in den Kanülenansatz integrierten mechanischen Sperrvorrichtung,
- Fig. 3: eine vergrößerte Detaildarstellung der Kanülenanordnung nach Fig. 2 mit Blickrichtung auf die Sperrvorrichtung in einer Freigabestellung und bei einer distal gerichteten Vorschubbewegung eines Führungsdrahts,
- Fig. 4: die Kanülenanordnung nach den Fig. 2 und 3 in einer der Fig. 3 entsprechenden Darstellungsweise in einer Sperrstellung der Sperrvorrichtung und bei einer proximal gerichteten Rückzugbewegung des Führungsdrahts,
- Fig. 5: in teilweise aufgeschnittener, schematischer Perspektivdarstellung eine Ausführungsform einer erfindungsgemäßen Kanülenanordnung mit einer in das Kanülenrohr integrierten Sperrvorrichtung,
- Fig. 6: eine vergrößerte perspektivische Detaildarstellung der Kanülenanordnung nach Fig. 5 im Bereich der Sperrvorrichtung,
- Fig. 7: eine weitere perspektivische und teilweise aufgeschnittene Detaildarstellung der Kanülenanordnung nach den Fig. 5 und 6 im Bereich der Sperrvorrichtung,
- Fig. 8: in teilweise aufgeschnittener, schematischer Perspektivdarstellung eine weitere Ausführungsform einer nicht beanspruchten Kanülenanordnung, deren Sperrvorrichtung zwei Sperrrollen umfasst,
- Fig. 9: die Kanülenanordnung nach Fig. 8 in einem weiteren Anwendungszustand und
- Fig. 10: die Kanülenanordnung nach den Fig. 8 und 9 in einer Sperrstellung der Sperrvorrichtung.

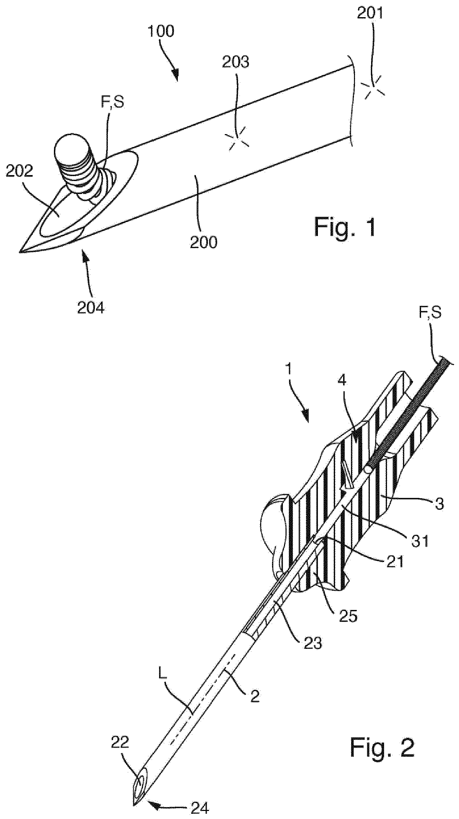

Anhand Fig. 1 ist bereichsweise und schematisch stark vereinfacht eine aus dem Stand der Technik bekannte Kanülenanordnung 100 mit einem längserstreckten Kanülenrohr 200 gezeigt. Das Kanülenrohr 200 weist eine nicht näher zeichnerisch dargestellte proximale Eintrittsöffnung 201, eine distale Austrittsöffnung 202, ein zwischen der Eintrittsöffnung 201 und der Austrittsöffnung 202 durchgängig längserstrecktes Lumen 203 und eine die Austrittsöffnung 202 berandende angeschärfte Kanülenspitze 204 auf. Die angeschärfte Kanülenspitze 204 erleichtert eine Punktion von tierischem oder menschlichem Körpergewebe und kann auch als Anschliff oder Kanülenauge bezeichnet werden. Weiter ist anhand Fig. 1 ein biegeflexibler medizinischer Funktionsstrang F in Gestalt eines Führungsdrahts S gezeigt. Der Führungsdraht S kann auch als Seldinger-Draht bezeichnet werden und weist eine dem Fachmann bekannte Funktionsweise und Gestaltung auf. Der Führungsdraht S ist einends in die Eintrittsöffnung 201 eingeführt, entlang des Lumens 203 vorgeschoben und ragt in der anhand Fig. 1 gezeigten Konfiguration stirnendseitig aus der Austrittsöffnung 202 heraus. Zudem ist der Führungsdraht S in einem stirnendseitig biegedeformierten Zustand gezeigt, wie er sich beispielsweise durch ein Anlaufen des Führungsdrahts S an ein gewebeseitiges Hindernis, wie beispielsweise eine Venenwand, eine Organwand oder dergleichen, einstellen kann.

Ausgehend von der anhand Fig. 1 gezeigten Konfiguration kann es bei einer in proximaler Richtung gerichteten Rückzugbewegung des Führungsdrahts S zu einer Beschädigung desselben durch die Kanülenspitze 204 kommen. Wird der Führungsdraht S zurückgezogen, wirkt die durch die Kanülenspitze 204 gebildete Berandung der Austrittsöffnung 202 nach Art einer Schneide auf den Führungsdraht S ein. Hierdurch kann der Führungsdraht S schlechtestenfalls durchtrennt werden, was mit schwerwiegenden medizinischen Komplikationen einhergehen kann.

Um solche Komplikationen zu vermeiden, weist die anhand der Fig. 2, 3 und 4 gezeigte Kanülenanordnung 1 eine mechanische Sperrvorrichtung 4 auf, die auf noch näher beschriebene Weise eine proximal gerichtete Rückzugbewegung des Funktionsstrangs F selbsttätig sperrt. Bevor auf die weiteren Einzelheiten und die Funktionsweise der mechanischen Sperrvorrichtung 4 im Detail eingegangen wird, werden zunächst weitere räumlich-körperliche und funktionelle Merkmale der Kanülenanordnung 1 erläutert:
Die Kanülenanordnung 1 weist ein längserstrecktes Kanülenrohr 2 mit einer proximalen Eintrittsöffnung 21 und einer distalen Austrittsöffnung 22 auf. Die proximale Eintrittsöffnung 21 und die distale Austrittsöffnung 22 sind mittels eines durchgängig längserstreckten Lumens 23 miteinander verbunden. Distal angeordnet weist das Kanülenrohr 2 eine angeschärfte Kanülenspitze 24 auf, welche die Austrittsöffnung 22 berandet. Das Kanülenrohr 2 ist zur Aufnahme und Führung des biegeflexiblen medizinischen Funktionsstrangs F, der anhand Fig. 2 wiederum als Führungsdraht S dargestellt ist, eingerichtet. Insoweit ist die Gestaltung des Kanülenrohrs 2 grundsätzlich übereinstimmend mit der Gestaltung des aus dem Stand der Technik bekannten Kanülenrohrs 200.

Weiter weist die Kanülenanordnung 1 einen Kanülenansatz 3 auf. Der Kanülenansatz 3 trägt das Kanülenrohr 2 und ist auf eine dem Fachmann bekannte Weise an ein der Kanülenspitze 24 abgewandtes Kanülenende 25 des Kanülenrohrs 2 angefügt. Der Kanülenansatz 3 ist zwischen einem nicht näher bezeichneten proximalen Ende und einem nicht näher bezeichneten distalen Ende längserstreckt und weist ein durchgängig längserstrecktes Lumen 31 auf, das einends in die Eintrittsöffnung 21 des Kanülenrohres 2 mündet. Das Lumen 31 des Kanülenansatzes 3 und das Lumen 23 des Kanülenrohrs 2 sind zueinander koaxial längserstreckt. Die übrige anhand Fig. 2 ersichtliche Formgebung des Kanülenansatzes 3 ist im Hinblick auf die vorliegende Erfindung nicht von vornehmlicher Bedeutung, so dass weitere diesbezügliche Erläuterungen entbehrlich sind.

Wie weiter anhand Fig. 2 gezeigt ist, ist der Führungsdraht S ausgehend von dem proximalen Ende des Kanülenansatzes 3 in dessen Lumen 31 und ausgehend von diesem über die Eintrittsöffnung 21 in das Lumen 23 des Kanülenrohrs 2 und von dort weiter bis in eine über die Austrittsöffnung 22 hinausgehende Konfiguration vorschiebbar. Die Vorschubbewegung erfolgt hierbei in distaler Richtung entlang einer Längsachse L des Lumens 23, die bei der gezeigten Ausführungsform koaxial mit der Längsachse des Kanülenrohrs und des Lumens 31 ist.

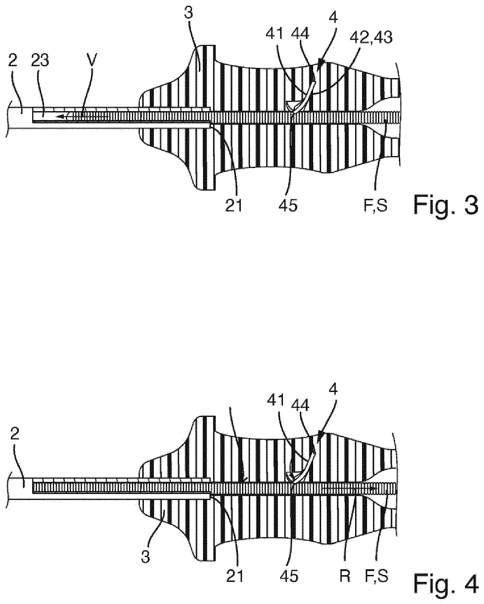

Die mechanische Sperrvorrichtung 4 ist bei der anhand der Fig. 2 bis 4 gezeigten Ausführungsform in den Kanülenansatz 3 integriert. Zudem weist die mechanische Sperrvorrichtung 4 ein dem Lumen 23 zugeordnetes Sperrelement 41 auf. Das Sperrelement 41 ist zum mechanischen Zusammenwirken mit dem Funktionsstrang F, genauer: des Führungsdrahts S, eingerichtet. Dabei wirkt das Sperrelement 41 mit einer Außenfläche M des Funktionsstrangs F zusammen, die auch als Mantelfläche bezeichnet werden kann. In Abhängigkeit der Bewegungsrichtung des Funktionsstrangs F ist das Sperrelement 41 selbsttätig überführbar zwischen einer Sperrstellung (Fig. 4) und einer Freigabestellung (Fig. 3). In der Sperrstellung sperrt das Sperrelement 41 eine proximal gerichtete Rückzugbewegung des Funktionsstrangs F. Hierzu wirkt das Sperrelement 41 kraft- und/oder formschlüssig auf die Außenfläche M ein. Dementgegen ist diese Einwirkung bzw. Sperrung in der Freigabestellung aufgehoben.

Die anhand der Fig. 3 und 4 ersichtlichen Pfeile symbolisieren eine distal gerichtete Vorschubbewegung V und eine proximal gerichtete Rückzugbewegung R.

Bei der gezeigten Ausführungsform ist das Sperrelement 41 eine Sperrklinke 42, die elastisch federbeweglich zwischen der Sperr- und der Freigabestellung verlagerbar ist. Die Sperrklinke 42 ist vorliegend eine separat von dem Kanülenansatz 3 gefertigte und hiernach in diesen integrierte Biegefeder 43. Die Biegefeder 43 ist einends, an einem radial außen liegenden Stirnende 44, in einer nicht näher bezeichneten Aufnahmeaussparung des Kanülenansatzes 3 festgelegt. Beispielsweise kann das Stirnende 44 kraft-, form- und/oder stoffschlüssig in der Aufnahmeaussparung festgelegt sein.

Bei einer zeichnerisch nicht näher dargestellten Ausführungsform ist die Sperrklinke 42 und/oder die Biegefeder 43 durch einen Abschnitt des Kanülenansatzes gebildet und insoweit einstückig zusammenhängend mit den übrigen Abschnitten des Kanülenansatzes.

Radial andernends weist die Sperrklinke 42 in Form der Biegefeder 43 eine Sperrkante 45 auf. Die Sperrkante 45 ist in distaler Richtung ausgerichtet. Bei der Vorschubbewegung V gleitet die Außenfläche M in distaler Richtung entlang der Sperrkante 45. Aufgrund der distalen Ausrichtung der Sperrkante 45 bewirkt die hierbei auftretende Reibungskraft eine radial nach außen gerichtete Kraftkomponente auf die Sperrkante 45. Dies wirkt einem Verklemmen der Sperrkante 45 an der Außenfläche M entgegen und gewährleistet eine leichtgängige Vorschubbewegung V.

Bei einer Rückzugbewegung R (Fig. 4) bewirkt die zwischen dem Funktionsstrang, genauer dessen Außenfläche M, und der Sperrkante 45 auftretende Reibungskraft eine in radialer Richtung nach innen orientierte Kraftkomponente. Diese radial nach innen gerichtete Kraftkomponente bewirkt, dass die Sperrkante 45 in radialer Richtung auf die Außenfläche M gepresst wird. Letztlich wird hierdurch ein Kraft- und/oder Formschluss mit der Außenfläche M und eine Sperrung der Rückzugbewegung R bewirkt.

Im Übrigen ist die Sperrklinke 42 und/oder die Biegefeder 43 bei der gezeigten Ausführungsform aus einem federelastischen Elastomerwerkstoff gefertigt. Denkbar ist zudem eine Fertigung aus Federstahl oder dergleichen.

Bei einer zeichnerisch nicht dargestellten Ausführungsform kann die Sperrklinke alternativ oder zusätzlich schwenkbeweglich zwischen der Freigabe- und der Sperrstellung verlagerbar sein. Dementsprechend ist nicht zwingend eine federelastische Gestaltung der Sperrklinke erforderlich. Stattdessen kann die Sperrklinke einends mittels eines Schwenklagers schwenkbeweglich an dem Katheteransatz oder dem Kanülenrohr gelagert sein.

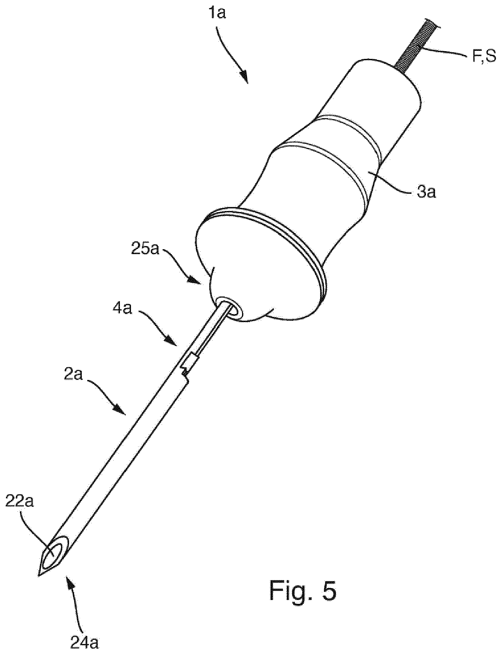

Anhand der Fig. 5 bis 7 ist eine Ausführungsform einer erfindungsgemäßen Kanülenanordnung 1a gezeigt. Nachfolgend wird lediglich auf wesentliche Unterschiede der Kanülenanordnung 1a gegenüber der Kanülenanordnung 1 nach den Fig. 2 bis 4 eingegangen. Wesentlicher Unterschied der Kanülenanordnung 1a ist, dass die mechanische Sperrvorrichtung 4a in das Kanülenrohr 2a integriert ist. Abgesehen von der Integration der Sperrvorrichtung 4a weist das Kanülenrohr 2a eine mit dem Kanülenrohr 2 der Kanülenanordnung 1 weitestgehend identische Gestaltung und/oder Funktion auf. Zudem ist an dem Kanülenende 25a des Kanülenrohrs 2a wiederum ein Kanülenansatz 3a befestigt. Der Kanülenansatz 3a weist - mit Ausnahme der nun nicht vorhandenen, integrierten mechanischen Sperrvorrichtung 4 - eine mit dem Kanülenansatz 3 identischen Aufbau auf. Weitere Erläuterungen zu dem Kanülenansatz 3a und der grundlegenden Gestaltung des Kanülenrohrs 2a sind daher entbehrlich.

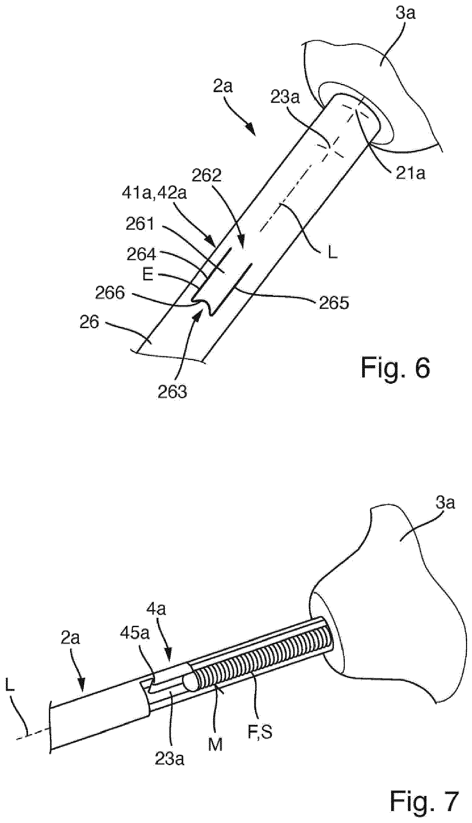

Auch bei der Ausführungsform nach den Fig. 5 bis 7 weist die mechanische Sperrvorrichtung 4a ein Sperrelement 41a in Form einer Sperrklinke 42a auf. Dabei ist die Sperrklinke 42a durch einen zungenförmig freigestellten Rohrwandabschnitt 261 einer Rohrwandung 26 des Kanülenrohrs 2a gebildet. Der Rohrwandabschnitt 261 ist mittels eines im Wesentlichen U-förmigen und radial durch die Rohrwandung 26 erstreckten Einschnitts von angrenzenden Bereichen der Rohrwandung 26 freigestellt. Dementsprechend weist der Rohrwandabschnitt 261 ein einstückig mit der übrigen Rohrwandung 26 verbundenes erstes Stirnende 262 und ein gegenüberliegendes zweites Stirnende 263 auf. Das zweite Stirnende 263 ist in radialer Richtung des Kanülenrohrs 2a relativbeweglich zu den übrigen Abschnitten der Rohrwandung 26. Das erste Stirnende 262 ist proximal angeordnet. Das zweite Stirnende 263 ist distal angeordnet. An seinem zweiten Stirnende 263 weist die Sperrklinke 42a bzw. der Rohrwandabschnitt 262 eine radial innenliegende Sperrkante 45a auf, die jedenfalls abschnittsweise anhand Fig. 7 gezeigt ist. Die Sperrkante 45a wirkt in einer der Sperrkante 45 entsprechenden Weise mit der Außenfläche M des Führungsdrahts S zusammen.

Im Übrigen sind einander gegenüberliegende Längskanten 264, 265 des zungenförmigen Rohrwandabschnitts 261 parallel zur Längsachse L des Lumens 23a erstreckt. In Querrichtung und damit in Umfangsrichtung des Kanülenrohrs 2a ist eine Querkante 266 zwischen den beiden Längskanten 264, 265 erstreckt. Die Querkante 266 weist eine proximale Einbuchtung auf, die dementsprechend auch bei der Sperrkante 45a vorhanden ist. Dies führt zu einem verbesserten Formschluss an der Außenfläche M.

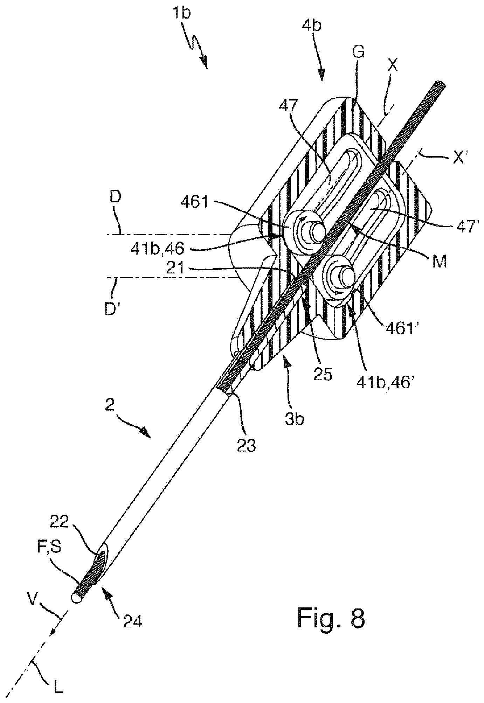

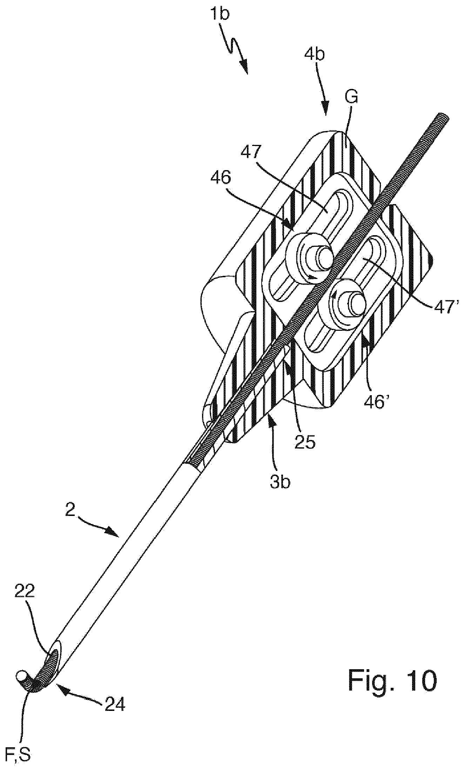

Eine weitere Ausführungsform einer Kanülenanordnung 1b ist anhand der Fig. 8 bis 10 gezeigt. Zur Vermeidung von Wiederholungen werden wiederum lediglich wesentliche Unterschiede der Kanülenanordnung 1b gegenüber den vorhergehenden Ausführungsformen erläutert.

Die Kanülenanordnung 1b weist wiederum ein Kanülenrohr 2 und einen Kanülenansatz 3b auf.

Das Kanülenrohr 2 ist in seiner Gestaltung und Funktion identisch mit dem Kanülenrohr 2 der Ausführungsform nach den Fig. 2 bis 4.

Der Kanülenansatz 3b ist wiederum proximal an das Kanülenrohr 2 angefügt.

Ein wesentlicher Unterschied ist, dass die Sperrvorrichtung 4b der Ausführungsform nach den Fig. 8 bis 10 weder in das Kanülenrohr 2 noch in den Kanülenansatz 3b integriert ist. Stattdessen weist die mechanische Sperrvorrichtung 4b ein Vorrichtungsgehäuse G auf, in welchem weitere Bauteile und/oder Abschnitte der Sperrvorrichtung 4b aufgenommen sind.

Im Übrigen versteht sich, dass das Vorrichtungsgehäuse G grundsätzlich auch dem Kanülenansatz 3b zugeordnet und insbesondere einstückig zusammenhängend mit diesem verbunden sein kann. Dessen ungeachtet ist das Vorrichtungsgehäuse G bei der gezeigten Ausführungsform auf eine zeichnerisch nicht näher dargestellte Weise form- und/oder kraftschlüssig lösbar proximal an dem Kanülenansatz 3b angefügt. Beispielsweise kann hierfür eine lösbare Rast-, Steck- oder Schraubverbindung vorgesehen sein.

Ein weiterer wesentlicher Unterschied liegt darin, dass die mechanische Sperrvorrichtung 4b anstelle einer Sperrklinke wenigstens eine Sperrrolle aufweist. Bei der gezeigten Ausführungsform sind eine erste Sperrrolle 46 und eine zweite Sperrrolle 46` vorhanden. Die erste Sperrrolle 46 und die zweite Sperrrolle 46` bilden ein Sperrrollenpaar. Die Gestaltung und Funktion der beiden Sperrrollen 46, 46` ist wenigstens weitestgehend identisch. Zudem sind die beiden Sperrrollen 46, 46' in Bezug auf die Längsachse L des Lumens 23 symmetrisch beidseits des Führungsdrahts S angeordnet. Zur Vermeidung von Wiederholungen wird nachfolgend daher in erster Linie auf die erste Sperrrolle 46 eingegangen. Die diesbezügliche Offenbarung gilt sinngemäß auch für die zweite Sperrrolle 46`, sofern nichts anderes erwähnt ist.

Die erste Sperrrolle 46 ist um eine Drehachse D drehbeweglich in dem Vorrichtungsgehäuse G gelagert. Die erste Sperrrolle 46 weist einen radial außen liegenden Außenumfang 461 auf, der zum Abwälzen auf der Außenfläche M eingerichtet ist. Dabei weist die erste Sperrrolle 46 eine mit der Vorschubbewegung V korrespondierende Freilaufdrehrichtung auf, die anhand des in Fig. 8 eingezeichneten Drehrichtungspfeils um die Drehachse D symbolisiert ist.

Weiter ist die erste Sperrrolle 46 entlang einer ersten Führungsachse X an einer Linearführung 47 linearbeweglich geführt verlagerbar. Die erste Führungsachse X ist relativ zu der Längsachse L in proximaler Richtung nach innen geneigt. Diese Neigung ist vergleichsweise gering ausgeführt und daher anhand der vorliegenden Figuren nicht ohne weiteres erkennbar. Die erste Linearführung 47 ist durch nicht näher bezeichnete Führungsspuren gebildet, die einander entlang der Drehachse D gegenüberliegend in dem Vorrichtungsgehäuse G angeordnet sind. Die Führungsspuren wirken mit nicht näher bezeichneten Führungszapfen der ersten Sperrrolle 46 zusammen. Die Führungszapfen sind insoweit entlang der ersten Führungsachse X gleitbeweglich und/oder rollbeweglich in den Führungsspuren aufgenommen und zudem um die - entlang der ersten Führungsachse X verschiebliche - Drehachse D drehbeweglich.

Die vorhergehenden Erläuterungen in Bezug auf die erste Sperrrolle 46 gelten sinngemäß entsprechend auch für die zweite Sperrrolle 46`. Der zweiten Sperrrolle 46` zugeordnete funktionsgleiche Bauteile und/oder Abschnitte der mechanischen Sperrvorrichtung 4b sind unter Verwendung identischer Bezugszeichenziffern und Hinzufügung eines Apostrophs gekennzeichnet. Insoweit sind insbesondere eine weitere bzw. zweite Drehachse D', eine zweite Führungsachse X' und eine zweite Linearführung 47` vorhanden.

Die weitere Funktionsweise der mechanischen Sperrvorrichtung 4b kann wie folgt beschrieben werden:
Bei einer Vorschubbewegung V wälzen die Außenumfänge 461, 461` auf der Außenfläche M ab. Dabei stellt sich jeweils die anhand der Drehbewegungspfeile der Fig. 8 symbolisierte Drehrichtung um die erste Drehachse D bzw. die zweite Drehachse D' ein. Diese Drehrichtungen sind jeweils als Freilaufdrehrichtungen aufzufassen. Die Sperrrollen 46, 46` nehmen hierbei eine in Bezug auf die jeweilige Linearführung 47 bzw. 47` distale Endlage ein. Dementsprechend sind die nicht näher bezeichneten Führungszapfen distal endseitig an den Linearführungen 47, 47` gehalten, so dass keine weitergehende distale Verlagerung der Sperrrollen 46, 46` entlang der jeweiligen Führungsachse X, X' erfolgen kann.

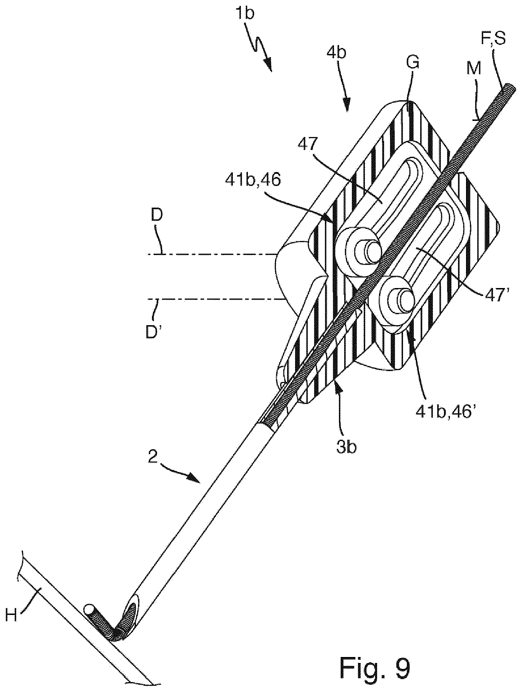

Anhand Fig. 9 ist eine Anwendungssituation der Kanülenanordnung 1b gezeigt, in welcher der Führungsdraht S an ein schematisch dargestelltes Hindernis H anstößt. In dieser Konfiguration ist üblicherweise eine proximale Rückzugbewegung des Führungsdrahts S erforderlich. Infolge der Rückzugbewegung werden die Sperrrollen 46, 46` zum einen in jeweils entgegengesetzter Richtung um die jeweilige Drehachse D, D' rotiert. Gleichzeitig erfolgt eine in proximaler Richtung gerichtete Verlagerung entlang der jeweiligen Führungsachse X, X'. Da die beiden Führungsachsen X, X' in proximaler Richtung radial geringfügig nach innen geneigt sind, werden die Sperrrollen 46, 46` infolge ihrer Linearbewegung gleichzeitig radial nach innen gegen den Führungsdraht S angestellt. Dies bewirkt ein verstärktes Anpressen des jeweiligen Außenumfangs 461, 461', was letztlich zu einer Sperrung der Rückzugbewegung führt.

Im Übrigen versteht sich, dass der medizinische Funktionsstrang F bzw. der Führungsdrahts S nicht notwendigerweise Bestandteil der Kanülenanordnungen 1, 1a, 1b ist.

## Patentansprüche

1. Kanülenanordnung (1a), aufweisend
ein längserstrecktes Kanülenrohr (2a) mit einer proximalen Eintrittsöffnung (21a), einer distalen Austrittsöffnung (22a), einem zwischen der Eintrittsöffnung (21a) und der Austrittsöffnung (22a) durchgängig längserstreckten Lumen (23a) und mit einer die Austrittsöffnung (22a) berandenden angeschärften Kanülenspitze (24a),
wobei das Kanülenrohr (2a) eingerichtet ist zur Aufnahme und Führung eines biegeflexiblen medizinischen Funktionsstrangs (F), der in das Lumen (23a) einführbar und entlang des Lumens (23a) beweglich ist,
wobei eine Sperrvorrichtung (4a) mit wenigstens einem dem Lumen (23a) zugeordneten Sperrelement (41a) vorhanden ist, wobei das wenigstens eine Sperrelement (41a) zum Zusammenwirken mit dem Funktionsstrang (F) eingerichtet und in Abhängigkeit einer Bewegungsrichtung des Funktionsstrangs (F) selbsttätig überführbar ist zwischen einer Sperrstellung, in welcher das wenigstens eine Sperrelement (41a) eine proximal gerichtete Rückzugbewegung (R) des Funktionsstrangs (F) unter Einwirkung auf den Funktionsstrang (F) sperrt, und einer Freigabestellung, in welcher das wenigstens eine Sperrelement (41a) eine distal gerichtete Vorschubbewegung (V) freigibt, **dadurch gekennzeichnet, dass** die Sperrvorrichtung (4a) in das Kanülenrohr (2a) integriert ist.

2. Kanülenanordnung (1a) nach Anspruch 1, **dadurch gekennzeichnet, dass** das wenigstens eine Sperrelement (41a) eine Sperrklinke (42a) ist, die elastisch federbeweglich und/oder schwenkbeweglich zwischen der Freigabestellung und der Sperrstellung verlagerbar ist und eine in distaler Richtung ausgerichtete Sperrkante (45a) aufweist, die bei einer Vorschubbewegung (V) gleitbeweglich mit einer Außenfläche (M) des Funktionsstrangs (F) zusammenwirkt und bei einer Rückzugbewegung (R) reibkraftbedingt gegen die Außenfläche (M) angestellt ist und die Rückzugbewegung (R) kraft- und/oder formschlüssig sperrt.

3. Kanülenanordnung (1a) nach Anspruch 2, **dadurch gekennzeichnet, dass** die Sperrklinke (42a) nach Art einer Biegefeder (43) gestaltet ist, wobei die Biegefeder (43) einends relativ zu dem Lumen (23) festgelegt ist und andernends die Sperrkante (45a) aufweist.

4. Kanülenanordnung (1a) nach Anspruch 2 oder 3, **dadurch gekennzeichnet, dass** die Sperrklinke (42a) durch einen zungenförmig freigestellten Rohrwandabschnitt (261) einer Rohrwandung (26) des Kanülenrohrs (2a) gebildet ist.

5. Kanülenanordnung (1a) nach einem der vorhergehenden Ansprüche, umfassend den medizinischen Funktionsstrang (F), insbesondere wobei der medizinische Funktionsstrang (F) ein medizinischer Führungsdraht (S), bevorzugt ein Seldinger-Draht, ist.

## Claims

1. A cannula assembly (1a), having
an elongate cannula tube (2a) with a proximal inlet opening (21a), a distal outlet opening (22a), a lumen (23a) extending continuously between the inlet opening (21a) and the outlet opening (22a), and with a sharpened cannula tip (24a) delimiting the outlet opening (22a),
wherein the cannula tube (2a) is configured to receive and guide a flexible medical functional section (F), which can be inserted into the lumen (23a) and is movable along the lumen (23a),
wherein a blocking device (4a) with at least one blocking element (41a) is provided and assigned to the lumen (23a), wherein the at least one blocking element (41a) is configured to interact with the flexible medical functional section (F) and, depending on a movement direction of the flexible medical functional section (F), is automatically transferred between a blocking position, in which the at least one blocking element (41a) blocks a proximally directed withdrawal movement (R) of the flexible medical functional section (F) by acting on the flexible medical functional section (F), and a release position, in which the at least one blocking element (41a) releases a distally directed advancing movement (V),
**characterized in that** the blocking device (4a) is integrated in the cannula tube (2a).

2. The cannula assembly (1a) as claimed in claim 1, wherein the at least one blocking element (41a) is a blocking pawl (42a) which is spring-elastically movable and/or pivotally movable between the release position and the blocking position and has a blocking edge (45a) which is oriented in the distal direction and, during an advancing movement (V), interacts slidably with an outer surface (M) of the flexible medical functional section (F) and, during a withdrawal movement (R), is caused by frictional force to be positioned against the outer surface (M) and blocks the withdrawal movement (R) with a force fit and/or form fit.

3. The cannula assembly (1a) as claimed in claim 2, wherein the blocking pawl (42a) is configured in the manner of a bending spring (43), wherein the bending spring (43) is secured at one end relative to the lumen (23) and at the other end has the blocking edge (45a).

4. The cannula assembly (1a) as claimed in claim 2 or 3, wherein the blocking pawl (42a) is formed by a tube wall portion (261), that is released in the form of a tongue, of a tube wall (26) of the cannula tube (2a).

5. The cannula assembly (1a) as claimed in one of the preceding claims, comprising the flexible medical functional section (F), in particular wherein the flexible medical functional section (F) is a medical guide wire (S), preferably a Seldinger wire.

## Revendications

1. Assemblage de canule (1a), comportant
un tube de canule allongé (2a) avec une ouverture d'entrée proximale (21a), une ouverture de sortie distale (22a), une lumière (23a) s'étendant continuellement entre l'ouverture d'entrée (21a) et l'ouverture de sortie (22a), et avec une pointe de canule aiguisée (24a) délimitant l'ouverture de sortie (22a),
dans lequel le tube de la canule (2a) est configuré pour recevoir et guider une section fonctionnelle médicale flexible (F), qui peut être insérée dans la lumière (23a) et est mobile le long de la lumière (23a),
dans lequel un dispositif de blocage (4a) avec au moins un élément de blocage (41a) est fourni et affecté à la lumière (23a), dans lequel au moins un élément de blocage (41a) est configuré pour interagir avec la section fonctionnelle médicale flexible (F) et, en fonction d'une direction de mouvement de la section fonctionnelle médicale flexible (F), est automatiquement transféré entre une position de blocage, dans laquelle le au moins un élément de blocage (41a) bloque un mouvement de retrait (R) en direction proximale de la section fonctionnelle médicale flexible (F) en agissant sur la section fonctionnelle médicale flexible (F), et une position de libération, dans laquelle le au moins un élément de blocage (41a) libère un mouvement d'avancement (V) en direction distale,
**caractérisé par le fait que** le dispositif de blocage (4a) est intégré dans le tube de la canule (2a).

2. Assemblage de canule (1a) selon la revendication 1, dans laquelle l'au moins un élément de blocage (41a) est un cliquet de blocage (42a) qui est mobile élastiquement et/ou pivotant entre la position de libération et la position de blocage et qui a un bord de blocage (45a) qui est orienté dans la direction distale et qui, pendant un mouvement d'avancement (V), interagit de manière coulissante avec une surface extérieure (M) de la section fonctionnelle médicale flexible (F) et, au cours d'un mouvement de retrait (R), est amené par une force de friction à se positionner contre la surface extérieure (M) et bloque le mouvement de retrait (R) par un ajustement forcé et/ou un ajustement de forme.

3. Assemblage de canule (1a) selon la revendication 2, dans laquelle le cliquet de blocage (42a) est configuré à la manière d'un ressort de flexion (43), dans lequel le ressort de flexion (43) est fixé à une extrémité par rapport à la lumière (23) et à l'autre extrémité a le bord de blocage (45a).

4. Assemblage de canule (1a) selon la revendication 2 ou 3, dans lequel le cliquet de blocage (42a) est formé par une partie de la paroi du tube (261), qui est libérée sous la forme d'une languette, d'une paroi du tube (26) du tube de la canule (2a).

5. Assemblage de canule (1a) selon l'une des revendications précédentes, comprenant la section fonctionnelle médicale flexible (F), en particulier dans laquelle la section fonctionnelle médicale flexible (F) est un fil de guidage médical (S), de préférence un fil de Seldinger.
